# EUROPÄISCHE PATENTSCHRIFT

(11) **EP 0 937 982 B1**
(45) Veröffentlichungstag und Bekanntmachung des Hinweises auf die Patenterteilung: **28.06.2006**
(21) Anmeldenummer: 99100403.7
(22) Anmeldetag: 09.01.1999
(51) Int. Cl.: G01N 33/576, G01N 33/564, C07K 14/47, C12N 15/12, A61K 39/00, A61M 1/38

(54) **Diagnostikum und Therapeutikum zur Behandlung der autoimmunen Hepatitis**
Diagnostic and therapeutic agent for treatment of autoimmune hepatitis
Agent diagnostique et thérapeutique pour le traitement de l'hépatite auto-immune

(30) Priorität: 13.02.1998 DE 19805815
(43) Veröffentlichungstag der Anmeldung: 25.08.1999
(73) Patentinhaber: Lohse, Ansgar W. Dr., 55129 Mainz (DE)
(72) Erfinder: Lohse, Ansgar W. Dr., 55129 Mainz (DE)
(74) Vertreter: UEXKÜLL & STOLBERG

(56) Entgegenhaltungen:
- DATABASE BIOSIS [Online] BIOSCIENCES INFORMATION SERVICE, PHILADELPHIA, PA, US; 1993 HEINTGES T ET AL: "Differentiation between autoimmune hepatitis and hepatitis C virus related liver disease." Database accession no. PREV199396049306 XP002192417 & ZEITSCHRIFT FUER GASTROENTEROLOGIE, Bd. 31, Nr. 5, 1993, Seiten 285-288, ISSN: 0044-2771
- DATABASE BIOSIS [Online] BIOSCIENCES INFORMATION SERVICE, PHILADELPHIA, PA, US; 1994 GRUBER R ET AL: "Detection of autoantibodies against M2, LKM-1, and SLA in liver diseases by standardized uniform ELISA-techniques." Database accession no. PREV199497459951 XP002192418 & JOURNAL OF CLINICAL LABORATORY ANALYSIS, Bd. 8, Nr. 5, 1994, Seiten 284-292, ISSN: 0887-8013
- DATABASE BIOSIS [Online] BIOSCIENCES INFORMATION SERVICE, PHILADELPHIA, PA, US; 1983 TEUFEL M ET AL: "CHRONIC ACTIVE HEPATITIS IN CHILDHOOD WITH DETECTION OF LIVER PANCREAS SPECIFIC AUTO ANTIBODIES" Database accession no. PREV198477003395 XP002192419 & EUROPEAN JOURNAL OF PEDIATRICS, Bd. 140, Nr. 1, 1983, Seiten 30-33, ISSN: 0340-6199
- MANNS M ET AL: "CHARACTERIZATION OF A NEW SUBGROUP OF AUTOIMMUNE CHRONIC ACTIVE HEPATITIS BY AUTOANTIBODIES AGAINST A SOLUBLE LIVER ANTIGEN" LANCET, Bd. 1, Nr. 8528, 1987, Seiten 292-294, XP002192416

## Beschreibung

Gegenstand der Erfindung ist ein Diagnostikum und ein Therapeutikum zur Behandlung der autoimmunen Hepatitis, bei dem durch eine Immunreaktion SLA/LP-Autoantikörper nachgewiesen werden.

Die autoimmune Hepatitis (AIH) ist eine chronische Leberentzündung, die unbehandelt zur Zirrhose führt, behandelt aber eine sehr gute Prognose hat. Eine rechtzeitige Diagnose ist daher wichtig. Es wird geschätzt, daß 5% aller Patienten mit einer chronischen Hepatitis in den westlichen Ländern eine AIH haben. Zur Zeit gibt es keinen spezifischen diagnostischen Test für die AIH, sondern die Diagnose beruht auf mehreren Kriterien wie dem Ausschluß einer Virushepatitis, dem Nachweis erhöhter Immunglobulingspiegel, dem Gewebetyp (HLA-Typ) und dem Nachweis von Autoantikörpern. Autoantikörper finden sich bei etwa 90% der Patienten, wobei die meisten nachweisbaren Autoantikörper zumindest in niedrigen Titern auch bei anderen Leberentzündungen auftreten können. Dies sind insbesondere Antikörper gegen nukleäre Antigene (ANA) und gegen die glatte Muskulatur (SMA) sowie die sehr seltenen Antikörper gegen Cytochrom p450 (LKM). 1987 wurden außerdem sog. SLA-Autoantikörper beschrieben (Manns M. et al., Lancet 1987;1:292-4), die nach den bisherigen Untersuchungen bei etwa 25 bis 30% der Patienten mit AIH vorkommen, aber - soweit bekannt - nicht (oder fast nicht) bei anderen Erkrankungen, auch nicht bei anderen Autoimmunerkrankungen (Lohse A.W. et al., Z. Gastroenterol. 1995;33:527-33). Spätere Untersuchungen haben darüber hinaus gezeigt, dass SLA-Antigene auch durch sog. LP-Antikörper (Liver-Pankreas-Antikörper) erkannt werden (Stechmesser et al.; Hepatology 1993; 18:1-8). Da SLA-Antikörper enthaltende Seren und LP-Antikörper enthaltende Seren in gleicher Weise mit SLA-Antigenen reagieren, sind beide Antikörpersysteme identisch. Deshalb wird im folgenden das Antigen als SLA/LP-Antigen bezeichnet. Der Nachweis von SLA/LP-Autoantikörpern ist also von hohem diagnostischen Wert für den Nachweis einer AIH.

Voraussetzung für die Entwicklung eines spezifischen Immunoassays zum Nachweis von SLA/LP-Antikörpern in Patientenseren ist die Identifizierung des SLA/LP-Antigens. Das ist bisher nicht gelungen. Zwar wurden bereits 1990 die Leber-Zytokeratine 8 und 18 als Zielantigene der SLA/LP-Autoantikörper beschrieben (Journal of Hepatology, 1990; 11: 232 bis 239), jedoch haben sich später diese Befunde nicht bestätigen und sogar widerlegen lassen (Wies I. et al., Z. Gastroenterol. 1998;36:93)

Es wurde nun ein Diagnostikum zum Erkennen einer autoimmunen Hepatitis gefunden, mit dem im Blutserum eines Patienten Antikörper gegen zwei Proteine nachgewiesen werden können, die die Aminosäuresequenz entsprechend der SEQ ID No: 2 oder der SEQ ID No. 4 des Sequenzprotokolls ganz oder teilweise enthalten und von SLA/LP-Auto-Antikörpern erkannt werden. Diese Proteine werden als SLA/LP-Antigen-1 und als SLA/LP-Antigen-2 bezeichnet. Teile oder natürliche oder künstliche Varianten dieser Antigene, die den im Sequenzprotokoll angegebenen Aminosäuresequenzen teilweise entsprechen und ebenfalls von SLA/LP-Auto-Antikörpern erkannt werden, sind gleichfalls Gegenstand der vorliegenden Erfindung.

Außerdem wurden für die genannten SLA/LP-Antigene auch die cDNAs gefunden, die eine Nukelotidsequenz entsprechend der SEQ ID No. 1 oder der SEQ ID No. 3 aufweisen. Diese cDNAs liegen als zwei splice-Varianten vor, von denen die längere (SEQ ID No. 3) ein Insert von 156 Nukleotiden hat. Diese beiden varianten erklären, warum SLA/LP-positive Seren im Western Blot eine Doppelbande zeigen. Die Doppelbande liegt bei 50 kDa, was dem Molekulargewicht der SLA/LP-Antigene entspricht. Bei einer Inkubation des SLA/LP-positiven Serums mit dem erfindungsgemäßen Fusionsprdtein verschwindet die Doppelbande des SLA/LP-Antigens im Western Blot, während ein den gleichen Vektor enthaltendes anderes Fusionsprotein die Doppelbande nicht beeinflußt. Gegenstand der Erfindung ist auch eine cDNA, die für einen Teil oder eine natürliche oder künstliche Variante eines der SLA/LP-Antigene kodiert, sofern diese cDNA wenigstens einen Teil der Nukleotidsequenz entsprechend der SEQ ID No. 1 oder der SEQ ID No. 3 aufweist und für ein Protein kodiert, das von SLA/LP-Auto-Antikörpern erkannt wird.

Mit Hilfe der erfindungsgemäßen cDNAs können die SLA/LP-Antigene rekombinant oder synthetisch in größeren Mengen erzeugt und nach bekannten Methoden der mittels der hierfür spezifischen SLA/LP-Auto-Antikörper gemessen-werden.

Damit stehen nunmehr alle Voraussetzungen zur Verfügung, um einen zuverlässigen und spezifischen Immunoassay zum Nachweis von SLA/LP-Autoantikörpern zu entwickeln. Für den Nachweis sind ein Radioimmunoassay (RIA), ein Chemolumineszenz-Immunoassay (CIA), ein Immunoblot-Assay oder ein Enzym-Immunoassay (EIA) in gleicher Weise geeignet. In allen Fällen wird eines der SLA/LP-Antigene an eine Matrix gebunden, z.B. eine Mikrotiterplatte oder eine Blot-Membran, und das zu testende Patientenserum hinzupipettiert. Nach Inkubation wird das Testserum abgewaschen und die spezifische Bindung von zu detektierenden SLA/LP-Autoantikörpern mittels eines mit einem Tracer markierten Sekundärantikörpers (anti-human Immunglobulin oder gegen eine Immunglobulin-Subklasse) nachgewiesen. Als Tracer kann z.B. eine Enzymverbindung wie Peroxidase oder Alkalische Phosphatase, eine radioaktive oder eine chemolumineszierende Substanz verwendet werden. Je mehr SLA/LP-Autoantikörper im Testserum vorhanden ist, desto stärker ist die Bindung an das Antigen. Als Standard wird zum einen Normalserum von gesunden Blutspendern (als Negativkontrolle) sowie ein Patientenserum mit SLA/LP-Antikörpern in unterschiedlichen Verdünnungen eingesetzt. Alternativ kann auch ein Inhibitionsimmunoassay mit den erfindungsgemäßen SLA/LP-Antigenen durchgeführt werden, in dem die Bindung eines definierten und mit einem Tracer versehenen SLA/LP-Autoantikörper enthaltenden Patientenserum inhibiert wird, sofern in dem Testserum ebenfalls SLA/LP-Autoantikörper vorhanden sind.

Da das SLA/LP-Antigen nur bei Patienten mit autoimmunen Lebererkrankungen immunologisch erkannt wird, hat es für die Immunpathogenese dieser Erkrankung eine wichtige Bedeutung. Es ist deshalb möglich, das SLA/LP-Antigen als spezifisches Immuntherapeutikum zu nutzen. Gegenstand der Erfindung ist deshalb auch ein Arzneimittel zur Behandlung der autoimmunen Hepatitis, das das genannte Antigen enthält. Es kann in spezifischen, immuntherapeutischen Verfahren eingesetzt werden, beispielsweise bei der Induktion einer oralen oder nasalen Toleranz, der T-Zell-Vakzination oder der extrakorporalen Immunabsorption. Durch die Identifizierung und Isolierung des erfindungsgemäßen SLA/LP-Antigens sind alle Voraussetzungen geschaffen, dieses Antigen zur Herstellung eines spezifischen Immuntherapeutikums bei der autoimmunen Hepatitis zu nutzen.

Die Erfindung wird durch das folgende Beispiel näher erläutert:

### Beispiel 1

### Nachweis von SLA/LP-Auto-Antikörpern im ELISA-Test unter Benutzung des rekombinanten SLA/LP-Antigens (SEQ ID No. 1)

Das rekombinante SLA/LP-Antigen wird mittels His-Tag aufgereinigt. 50 µg des SLA/LP-Antigens werden in 50 µl PBS in eine Mikrotiterplatte gegeben und über Nacht stehengelassen. Nach dem Abpipettieren und Waschen der Mikrotiterplatte wird ein post-coating für eine Stunde mit einer 1%igen BSA/PBS-Lösung bei Raumtemperatur durchgeführt. Danach wurden jeweils 50 µl von Normalseren, Patientenseren und Kontrollseren im Verhältnis 1:100 verdünnt und in einer Menge von jeweils 50 µl 30 min auf der Mikrotiterplatte inkubiert. Nach dem Waschen erfolgte eine Inkubation mit einem Peroxidase-markierten Sekundär-Antikörper, dem antihuman Immunglobulin, das im Verhältnis 1:8000 verdünnt war. Nach dem Auswaschen des überschüssigen Sekundär-Antikörpers wurde eine ABTS-Lösung [ 55 mg der 2,2'-Azidodi-3-ethyl-benzthiazin-6-sulfonsäure(Serva,Heidelberg) in 5 ml 0,01 M K₂ HPO₄ (pH 6,0) , enthaltend 50 µl H₂O₂, verdünnt im Verhältnis 1:300 in PBS] hinzugefügt. Dabei wurden folgende Meßergebnisse erzielt:

| Serum | OD |
|---|---|
| Normalserum 1 | 0,476 |
| Normalserum 1 | 0,471 |
| SLA-Serum 1 | 1,863 |
| SLA-Serum 2 | 1,995 |
| SLA-Serum 3 | 1,791 |
| SLA-Serum 4 | 1,867 |
| AMA-pos. Serum (PBC) | 0,615 |
| LKM-pos. Serum (Hep. C) | 0,769 |

### Erläuterungen:

- OD =: Optical Density (Extinktion)
- AMA =: Anti-mitochondriale Antikörper
- PBC =: primary biliary cirrhosis
- LKM =: liver-kidney-microsomal
- BSA =: bovine serum albumin
- PBS =: phosphate-buffered saline

Alle das SLA/LP-Antigen enthaltenden Patientenseren zeigten bei der Messung in einem Titertec Multiscan MC (Flow Laboratories, Meckenheim) deutlich erhöhte Absorptionswerte verglichen mit dem Normalserum und verglichen mit dem Serum von Patienten, die an anderen Lebererkrankungen litten.

## Patentansprüche

1. Diagnostikum zum Erkennen der autoimmunen Hepatitis, mit dem im Blutserum eines Patienten Antikörper gegen Antigene nachgewiesen werden können, die die Aminosäuresequenz entsprechend der SEQ ID NO.2 oder der SEQ ID NO.4 des Sequenzprotokolls enthalten und von SLA/LP-Autoantikörpern erkannt werden, **dadurch gekennzeichnet, daß** es ein Protein umfaßt, welches die Aminosäuresequenz entsprechend der SEQ ID NO.2 oder der SEQ ID NO.4 des Sequenzprotokolls enthält und von SLA/LP-Autoantikörpern erkannt wird.

2. Verwendung eines Proteins, wobei das Protein die Aminosäuresequenz entsprechend der SEQ ID NO.2 oder der SEQ ID NO.4 des Sequenzprotokolls enthält und von SLA/LP-Autoantikörpern erkannt wird, zum Erkennen der autoimmunen Hepatitis *in vitro.*

3. Verwendung einer cDNA für die rekombinante Erzeugung eines Proteins zum Erkennen der autoimmunen Hepatitis, wobei das Protein die Aminosäuresequenz entsprechend der SEQ ID NO.2 oder der SEQ ID NO.4 des Sequenzprotokolls enthält und von SLA/LP-Autoantikörpern erkannt wird, **dadurch gekennzeichnet, daß** die cDNA die Nukleotidsequenz entsprechend der SEQ ID NO.1 oder der SEQ ID NO.3 aufweist.

4. Verfahren zum Nachweis von SLA/LP-Antikörpern, **dadurch gekennzeichnet, daß** man ein Protein, wobei das Protein die Aminosäuresequenz entsprechend der SEQ ID NO.2 oder der SEQ ID NO.4 des Sequenzprotokolls enthält und von SLA/LP-Autoantikörpern erkannt wird, an eine Matrix bindet und dieses mit dem auf SLA/LP-Autoantikörper zu untersuchenden Patientenserum und einem geeigneten Tracer inkubiert, anschließend den nicht-gebundenen Tracer entfernt und dann die Menge des durch das SLA/LP-Antigen gebundenen Tracers bestimmt.

5. Kit zur Durchführung eines Nachweisverfahrens nach Anspruch 4 umfassend ein Protein, wobei das Protein die Aminosäuresequenz entsprechend der SEQ ID NO.2 oder der SEQ ID NO.4 des Sequenzprotokolls enthält und von SLA/LP-Autoantikörpern erkannt wird.

6. Arzneimittel zur Behandlung der autoimmunen Hepatitis, **dadurch gekennzeichnet, daß** es ein Protein enthält, welches die Aminosäuresequenz entsprechend der SEQ ID NO.2 oder der SEQ ID NO.4 des Sequenzprotokolls enthält und von SLA/LP-Autoantikörpern erkannt wird.

7. Verwendung eines Proteins, welches die Aminosäuresequenz entsprechend der SEQ ID NO.2 oder der SEQ ID NO.4 des Sequenzprotokolls enthält und von SLA/LP-Autoantikörpern erkannt wird, zur Herstellung eines Arzneimittels zur Behandlung der autoimmunen Hepatitis.

8. Verwendung nach Anspruch 7, **dadurch gekennzeichnet, daß** man das Arzneimittel zur extrakorporalen Immunabsorption einsetzt.

## Claims

1. Diagnostic agent for diagnosing autoimmune hepatitis, with which it is possible to detect in a patient's blood serum antibodies against antigens which comprise the amino acid sequence corresponding to SEQ ID NO. 2 or to SEQ ID NO. 4 in the sequence listing and are recognized by SLA/LP autoantibodies, **characterized in that** it includes a protein which comprises the amino acid sequence corresponding to SEQ ID NO. 2 or to SEQ ID NO. 4 in the sequence listing, and is recognized by SLA/LP autoantibodies.

2. Use of a protein, where the protein comprises the amino acid sequence corresponding to SEQ ID NO. 2 or to SEQ ID NO. 4 in the sequence listing, and is recognized by SLA/LP autoantibodies, for diagnosing autoimmune hepatitis *in vitro.*

3. Use of a cDNA for recombinant production of a protein for diagnosing autoimmune hepatitis, where the protein comprises the amino acid sequence corresponding to SEQ ID NO. 2 or to SEQ ID NO. 4 in the sequence listing, and is recognized by SLA/LP autoantibodies, **characterized in that** the cDNA has the nucleotide sequence corresponding to SEQ ID NO. 1 or to SEQ ID NO. 3.

4. Method for detecting SLA/LP antibodies, **characterized in that** a protein, where the protein comprises the amino acid sequence corresponding to SEQ ID NO. 2 or to SEQ ID NO. 4 in the sequence listing, and is recognized by SLA/LP autoantibodies, is bound to a matrix and is incubated with the patient's serum which is to be investigated for SLA/LP autoantibodies, and with a suitable tracer, subsequently the unbound tracer is removed and then the amount of tracer bound by the SLA/LP antigen is determined.

5. Kit for carrying out a detection method according to Claim 4 comprising a protein, where the protein comprises the amino acid sequence corresponding to SEQ ID NO. 2 or to SEQ ID NO. 4 in the sequence listing, and is recognized by SLA/LP autoantibodies.

6. Medicament for the treatment of autoimmune hepatitis, **characterized in that** it comprises a protein which comprises the amino acid sequence corresponding to SEQ ID NO. 2 or to SEQ ID NO. 4 in the sequence listing, and is recognized by SLA/LP autoantibodies.

7. Use of a protein which comprises the amino acid sequence corresponding to SEQ ID NO. 2 or to SEQ ID NO. 4 in the sequence listing, and is recognized by SLA/LP autoantibodies, for producing a medicament for the treatment of autoimmune hepatitis.

8. Use according to Claim 7, **characterized in that** the medicament is employed for extracorporeal immunoabsorption.

## Revendications

1. Agent de diagnostic pour la détection de l'hépatite auto-immune, avec lequel il est possible de détecter dans le sérum sanguin d'un patient des anticorps dirigés contre des antigènes qui contiennent la séquence d'acides aminés correspondant à la séquence SEQ ID n°2 ou à la séquence SEQ ID n°4 du listage des séquences et sont détectés par les autoanticorps anti-SLA/LP, **caractérisé en ce qu'**il comprend une protéine, laquelle contient la séquence d'acides aminés correspondant à la séquence SEQ ID n°2 ou à la séquence SEQ ID n°4 du listage des séquences et est détectée par les autoanticorps anti-SLA/LP.

2. Utilisation d'une protéine, ladite protéine contenant la séquence d'acides aminés correspondant à la séquence SEQ ID n°2 ou à la séquence SEQ ID n°4 du listage des séquences et étant détectée par les autoanticorps anti-SLA/LP, pour détecter l'hépatite auto-immune *in vitro*.

3. Utilisation d'un ADNc pour la production recombinante d'une protéine destinée à détecter l'hépatite auto-immune, où la protéine contient la séquence d'acides aminés correspondant à la séquence SEQ ID n°2 ou à la séquence SEQ ID n°4 du listage des séquences et est détectée par les autoanticorps anti-SLA/LP, **caractérisée en ce que** l'ADNc comprend la séquence de nucléotides correspondant à la séquence SEQ ID n°1 ou à la séquence SEQ ID n°3.

4. Procédé de détection d'anticorps anti-SLA/LP, **caractérisé en ce qu'**on lie une protéine, ladite protéine contenant la séquence d'acides aminés correspondant à la séquence SEQ ID n°2 ou à la séquence SEQ ID n°4 du listage des séquences et étant détectée par les autoanticorps anti-SLA/LP, à une matrice et on incube celle-ci avec le sérum du patient dans lequel on recherche les autoanticorps anti-SLA/LP et un traceur adapté, puis on retire le traceur non lié et on déterminet ensuite la quantité du traceur lié par l'antigène SLA/LP.

5. Kit pour la mise en oeuvre d'un procédé de détection selon la revendication 4 comprenant une protéine, ladite protéine contenant la séquence d'acides aminés correspondant à la séquence SEQ ID n°2 ou à la séquence SEQ ID n°4 du listage des séquences et étant détectée par les autoanticorps anti-SLA/LP.

6. Médicament destiné à traiter l'hépatite auto-immune, **caractérisé en ce qu'**il contient une protéine, laquelle contient la séquence d'acides aminés correspondant à la séquence SEQ ID n°2 ou à la séquence SEQ ID n°4 du listage des séquences et est détectée par les autoanticorps anti-SLA/LP.

7. Utilisation d'une protéine, laquelle contient la séquence d'acides aminés correspondant à la séquence SEQ ID n°2 ou à la séquence SEQ ID n°4 du listage des séquences et est détectée par les autoanticorps anti-SLA/LP, pour fabriquer un médicament destiné à traiter l'hépatite auto-immune.

8. Utilisation selon la revendication 7, **caractérisée en ce que** le médicament est utilisé pour l'immunoabsorption extracorporelle.


### SEQUENZPROTOKOLL

<110> Privatdozent Dr. Lohse, Ansgar W.
<120> Diagnostikum zum Erkennen der autoimmunen Hepatitis
<130> L32P1EP
<140>
   <141>
<160> 4
<170> PatentIn Ver. 2.1
<210> 1
   <211> 1335
   <212> DNA
   <213> Homo sapiens
<220>
   <221> CDS
   <222> (1)..(1335)
<400> 1
   tcg cgg cgg gag agc ggc tgg tgt cgc cgg ctt acg tgc ggc agg gct    48
   Ser Arg Arg Glu Ser Gly Trp Cys Arg Arg Leu Thr Cys Gly Arg Ala
     1               5                  10                  15
   gtg agg ccc gcc gct cgc atg agc acc tca tac ggc tgc ttc tgg aga    96
   Val Arg Pro Ala Ala Arg Met Ser Thr Ser Tyr Gly Cys Phe Trp Arg
                20                  25                  30
   agg ttc att cat ggc att gga cga tcc ggt gat att tct gct gtg caa    144
   Arg Phe Ile His Gly Ile Gly Arg Ser Gly Asp Ile Ser Ala Val Gln
            35                  40                  45
   cca aaa gct gca ggc tct agc ctt ttg aac aaa att acc aat tct ttg    192
   Pro Lys Ala Ala Gly Ser Ser Leu Leu Asn Lys Ile Thr Asn Ser Leu
        50                  55                  60
   gtc ctg gac att ata aag ctg gct ggt gtc cat aca gta gcc aac tgc    240
   Val Leu Asp Ile Ile Lys Leu Ala Gly Val His Thr Val Ala Asn Cys
    65                  70                  75                  80
   ttt gta gtt cct atg gca act ggt atg agt cta act ctg tgt ttc tta    288
   Phe Val Val Pro Met Ala Thr Gly Met Ser Leu Thr Leu Cys Phe Leu
                    85                  90                  95
   aca tta cga cac aaa aga cca aag gca aag tat att ata tgg cca cga    336
   Thr Leu Arg His Lys Arg Pro Lys Ala Lys Tyr Ile Ile Trp Pro Arg
               100                 105                 110
   ata gac cag aag tcc tgc ttt aaa tcc atg atc act gca ggt ttt gag    384
   Ile Asp Gln Lys Ser Cys Phe Lys Ser Met Ile Thr Ala Gly Phe Glu
           115                 120                 125
   cct gtg gtg ata gaa aat gtt ttg gaa ggt gac gag ctg cgt aca gac    432
   Pro Val Val Ile Glu Asn Val Leu Glu Gly Asp Glu Leu Arg Thr Asp
       130                 135                 140
   ctg aaa gca gtg gag gct aaa gtc cag gaa ctt ggg cct gat tgc att    480
   Leu Lys Ala Val Glu Ala Lys Val Gln Glu Leu Gly Pro Asp Cys Ile
   145                 150                 155                 160
   ctg tgt att cat tct act aca tcc tgt ttt gct cca agg gtg cct gat    528
   Leu Cys Ile His Ser Thr Thr Ser Cys Phe Ala Pro Arg Val Pro Asp
                   165                 170                 175
   aga tta gaa gaa ctg gct gtg att tgt gct aat tat gac att cca cat    576
   Arg Leu Glu Glu Leu Ala Val Ile Cys Ala Asn Tyr Asp Ile Pro His
               180                 185                 190
   ata gtt aat aat gct tat gga gtg cag tct tca aag tgt atg cat ctc    624
   Ile Val Asn Asn Ala Tyr Gly Val Gln Ser Ser Lys Cys Met His Leu
           195                 200                 205
   att cag cag ggg gct cga gtt ggt aga ata gat gct ttt gtt cag agc    672
   Ile Gln Gln Gly Ala Arg Val Gly Arg Ile Asp Ala Phe Val Gln Ser
       210                 215                 220
   ttg gac aaa aat ttt atg gtt cca gta ggt ggt gct ata att gct ggc    720
   Leu Asp Lys Asn Phe Met Val Pro Val Gly Gly Ala Ile Ile Ala Gly
   225                 230                 235                 240
   ttt aat gat tca ttc att cag gaa atc agc aag atg tat cca gga aga    768
   Phe Asn Asp Ser Phe Ile Gln Glu Ile Ser Lys Met Tyr Pro Gly Arg
                   245                 250                 255
   gct tca gct tca cct tct tta gat gtc ctt att act tta ttg tca ctt    816
   Ala Ser Ala Ser Pro Ser Leu Asp Val Leu Ile Thr Leu Leu Ser Leu
               260                 265                 270
   gga tca aat ggc tat aag aag cta cta aaa gaa aga aag gaa atg ttt    864
   Gly Ser Asn Gly Tyr Lys Lys Leu Leu Lys Glu Arg Lys Glu Met Phe
           275                 280                 285
   tca tat ttg tcc aac caa ata aag aag ttg tca gaa gcc tac aat gaa    912
   Ser Tyr Leu Ser Asn Gln Ile Lys Lys Leu Ser Glu Ala Tyr Asn Glu
       290                 295                 300
   aga ctg ttg cat aca cct cac aat ccc ata tct tta gct atg aca ctt    960
   Arg Leu Leu His Thr Pro His Asn Pro Ile Ser Leu Ala Met Thr Leu
   305                 310                 315                 320
   aaa aca cta gat gaa cac cgt gac aaa gct gtc act cag ctt ggc tcg   1008
   Lys Thr Leu Asp Glu His Arg Asp Lys Ala Val Thr Gln Leu Gly Ser
                   325                 330                 335
   atg ctt ttt acc aaa cag gtt tct gga gcc agg gtt gtg cct ctt ggg   1056
   Met Leu Phe Thr Lys Gln Val Ser Gly Ala Arg Val Val Pro Leu Gly
               340                 345                 350
   tcc atg caa act gtg agt ggc tat act ttc aga ggc ttt atg tca cat   1104
   Ser Met Gln Thr Val Ser Gly Tyr Thr Phe Arg Gly Phe Met Ser His
           355                 360                 365
   aca aat aat tac cct tgt gct tac ctc aat gct gca tca gcc atc gga   1152
   Thr Asn Asn Tyr Pro Cys Ala Tyr Leu Asn Ala Ala Ser Ala Ile Gly
       370                 375                 380
   atg aar atg cag gat gtg gac ctg ttc ata aac ara ctt gac agg tgt   1200
   Met Lys Met Gln Asp Val Asp Leu Phe Ile Asn Xaa Leu Asp Arg Cys
   385                 390                 395                 400
   tta aag gca gta aga aaa gaa cga agt aaa gag agt gat gac aat tat      1248
   Leu Lys Ala Val Arg Lys Glu Arg Ser Lys Glu Ser Asp Asp Asn Tyr
                   405                 410                 415
   gac aaa act gaa rat gtg gat att gaa gaa atg gct tta aaa cta gat      1296
   Asp Lys Thr Glu Xaa Val Asp Ile Glu Glu Met Ala Leu Lys Leu Asp
               420                 425                 430
   aat gta ctt ctt gac aca tac cag gat gct tct tca tga                  1335
   Asn Val Leu Leu Asp Thr Tyr Gln Asp Ala Ser Ser
           435                 440                 445
<210> 2
   <211> 444
   <212> PRT
   <213> Homo sapiens
<400> 2
   Ser Arg Arg Glu Ser Gly Trp Cys Arg Arg Leu Thr Cys Gly Arg Ala
    1               5                  10                  15
   Val Arg Pro Ala Ala Arg Met Ser Thr Ser Tyr Gly Cys Phe Trp Arg
               20                  25                  30
   Arg Phe Ile His Gly Ile Gly Arg Ser Gly Asp Ile Ser Ala Val Gln
           35                  40                  45
   Pro Lys Ala Ala Gly Ser Ser Leu Leu Asn Lys Ile Thr Asn Ser Leu
       50                  55                  60
   Val Leu Asp Ile Ile Lys Leu Ala Gly Val His Thr Val Ala Asn Cys
   65                  70                  75                  80
   Phe Val Val Pro Met Ala Thr Gly Met Ser Leu Thr Leu Cys Phe Leu
                   85                  90                  95
   Thr Leu Arg His Lys Arg Pro Lys Ala Lys Tyr Ile Ile Trp Pro Arg
               100                 105                 110
   Ile Asp Gln Lys Ser Cys Phe Lys Ser Met Ile Thr Ala Gly Phe Glu
           115                 120                 125
   Pro Val Val Ile Glu Asn Val Leu Glu Gly Asp Glu Leu Arg Thr Asp
       130                 135                 140
   Leu Lys Ala Val Glu Ala Lys Val Gln Glu Leu Gly Pro Asp Cys Ile
   145                 150                 155                 160
   Leu Cys Ile His Ser Thr Thr Ser Cys Phe Ala Pro Arg Val Pro Asp
                   165                 170                 175
   Arg Leu Glu Glu Leu Ala Val Ile Cys Ala Asn Tyr Asp Ile Pro His
               180                 185                 190
   Ile Val Asn Asn Ala Tyr Gly Val Gln Ser Ser Lys Cys Met His Leu
           195                 200                 205
   Ile Gln Gln Gly Ala Arg Val Gly Arg Ile Asp Ala Phe Val Gln Ser
       210                 215                 220
   Leu Asp Lys Asn Phe Met Val Pro Val Gly Gly Ala Ile Ile Ala Gly
   225                 230                 235                 240
   Phe Asn Asp Ser Phe Ile Gln Glu Ile Ser Lys Met Tyr Pro Gly Arg
                   245                 250                 255
   Ala Ser Ala Ser Pro Ser Leu Asp Val Leu Ile Thr Leu Leu Ser Leu
               260                 265                 270
   Gly Ser Asn Gly Tyr Lys Lys Leu Leu Lys Glu Arg Lys Glu Met Phe
           275                 280                 285
   Ser Tyr Leu Ser Asn Gln Ile Lys Lys Leu Ser Glu Ala Tyr Asn Glu
       290                 295                 300
   Arg Leu Leu His Thr Pro His Asn Pro Ile Ser Leu Ala Met Thr Leu
   305                 310                 315                 320
   Lys Thr Leu Asp Glu His Arg Asp Lys Ala Val Thr Gln Leu Gly Ser
                   325                 330                 335
   Met Leu Phe Thr Lys Gln Val Ser Gly Ala Arg Val Val Pro Leu Gly
               340                 345                 350
   Ser Met Gln Thr Val Ser Gly Tyr Thr Phe Arg Gly Phe Met Ser His
           355                 360                 365
   Thr Asn Asn Tyr Pro Cys Ala Tyr Leu Asn Ala Ala Ser Ala Ile Gly
       370                 375                 380
   Met Lys Met Gln Asp Val Asp Leu Phe Ile Asn Xaa Leu Asp Arg Cys
   385                 390                 395                 400
   Leu Lys Ala Val Arg Lys Glu Arg Ser Lys Glu Ser Asp Asp Asn Tyr
                   405                 410                 415
   Asp Lys Thr Glu Xaa Val Asp Ile Glu Glu Met Ala Leu Lys Leu Asp
               420                 425                 430
   Asn Val Leu Leu Asp Thr Tyr Gln Asp Ala Ser Ser
           435                 440
<210> 3
   <211> 1491
   <212> DNA
   <213> Homo sapiens
<220>
   <221> CDS
   <222> (1)..(1491)
<400> 3
   tcg cgg cgg gag agc ggc tgg tgt cgc cgg ctt acg tgc ggc agg gct      48
   Ser Arg Arg Glu Ser Gly Trp Cys Arg Arg Leu Thr Cys Gly Arg Ala
     1               5                  10                  15
   gtg agg ccc gcc gct cgc atg agc acc tca tac ggc tgc ttc tgg aga      96
   Val Arg Pro Ala Ala Arg Met Ser Thr Ser Tyr Gly Cys Phe Trp Arg
               20                  25                  30
   agg gcw wwk tgt cca aag aat ggc tgg gat gaa agt aca ctt gaa ctc      144
   Arg Xaa Xaa Cys Pro Lys Asn Gly Trp Asp Glu Ser Thr Leu Glu Leu
           35                  40                  45
   ttt tta cat gaa ctt gca atc atg gac agc aac aat ttc tta ggc aat      192
   Phe Leu His Glu Leu Ala Ile Met Asp Ser Asn Asn Phe Leu Gly Asn
       50                  55                  60
   tgt ggt gtg gga gaa agg gaa ggg aga gtg gca tcc gca ctg gtt gct      240
   Cys Gly Val Gly Glu Arg Glu Gly Arg Val Ala Ser Ala Leu Val Ala
    65                  70                  75                  80
   cgt cgt cat tac agg ttc att cat ggc att gga cga tcc ggt gat att      288
   Arg Arg His Tyr Arg Phe Ile His Gly Ile Gly Arg Ser Gly Asp Ile
                    85                  90                  95
   tct gct gtg caa cca aaa gct gca ggc tct agc ctt ttg aac aaa att      336
   Ser Ala Val Gln Pro Lys Ala Ala Gly Ser Ser Leu Leu Asn Lys Ile
               100                 105                 110
   acc aat tct ttg gtc ctg gac att ata aag ctg gct ggt gtc cat aca      384
   Thr Asn Ser Leu Val Leu Asp Ile Ile Lys Leu Ala Gly Val His Thr
           115                 120                 125
   gta gcc aac tgc ttt gta gtt cct atg gca act ggt atg agt cta act      432
   Val Ala Asn Cys Phe Val Val Pro Met Ala Thr Gly Met Ser Leu Thr
       130                 135                 140
   ctg tgt ttc tta aca tta cga cac aaa aga cca aag gca aag tat att      480
   Leu Cys Phe Leu Thr Leu Arg His Lys Arg Pro Lys Ala Lys Tyr Ile
   145                 150                 155                 160
   ata tgg cca cga ata gac cag aag tcc tgc ttt aaa tcc atg atc act      528
   Ile Trp Pro Arg Ile Asp Gln Lys Ser Cys Phe Lys Ser Met Ile Thr
                   165                 170                 175
   gca ggt ttt gag cct gtg gtg ata gaa aat gtt ttg gaa ggt gac gag      576
   Ala Gly Phe Glu Pro Val Val Ile Glu Asn Val Leu Glu Gly Asp Glu
               180                 185                 190
   ctg cgt aca gac ctg aaa gca gtg gag gct aaa gtc cag gaa ctt ggg      624
   Leu Arg Thr Asp Leu Lys Ala Val Glu Ala Lys Val Gln Glu Leu Gly
           195                 200                 205
   cct gat tgc att ctg tgt att cat tct act aca tcc tgt ttt gct cca      672
   Pro Asp Cys Ile Leu Cys Ile His Ser Thr Thr Ser Cys Phe Ala Pro
       210                 215                 220
   agg gtg cct gat aga tta gaa gaa ctg gct gtg att tgt gct aat tat      720
   Arg Val Pro Asp Arg Leu Glu Glu Leu Ala Val Ile Cys Ala Asn Tyr
   225                 230                 235                 240
   gac att cca cat ata gtt aat aat gct tat gga gtg cag tct tca aag      768
   Asp Ile Pro His Ile Val Asn Asn Ala Tyr Gly Val Gln Ser Ser Lys
                   245                 250                 255
   tgt atg cat ctc att cag cag ggg gct cga gtt ggt aga ata gat gct      816
   Cys Met His Leu Ile Gln Gln Gly Ala Arg Val Gly Arg Ile Asp Ala
               260                 265                 270
   ttt gtt cag agc ttg gac aaa aat ttt atg gtt cca gta ggt ggt gct      864
   Phe Val Gln Ser Leu Asp Lys Asn Phe Met Val Pro Val Gly Gly Ala
           275                 280                 285
   ata att gct ggc ttt aat gat tca ttc att cag gaa atc agc aag atg      912
   Ile Ile Ala Gly Phe Asn Asp Ser Phe Ile Gln Glu Ile Ser Lys Met
       290                 295                 300
   tat cca gga aga gct tca gct tca cct tct tta gat gtc ctt att act      960
   Tyr Pro Gly Arg Ala Ser Ala Ser Pro Ser Leu Asp Val Leu Ile Thr
   305                 310                 315                 320
   tta ttg tca ctt gga tca aat ggc tat aag aag cta cta aaa gaa aga    1008
   Leu Leu Ser Leu Gly Ser Asn Gly Tyr Lys Lys Leu Leu Lys Glu Arg
                   325                 330                 335
   aag gaa atg ttt tca tat ttg tcc aac caa ata aag aag ttg tca gaa    1056
   Lys Glu Met Phe Ser Tyr Leu Ser Asn Gln Ile Lys Lys Leu Ser Glu
               340                 345                 350
   gcc tac aat gaa aga ctg ttg cat aca cct cac aat ccc ata tct tta    1104
   Ala Tyr Asn Glu Arg Leu Leu His Thr Pro His Asn Pro Ile Ser Leu
           355                 360                 365
   gct atg aca ctt aaa aca cta gat gaa cac cgt gac aaa gct gtc act    1152
   Ala Met Thr Leu Lys Thr Leu Asp Glu His Arg Asp Lys Ala Val Thr
       370                 375                 380
   cag ctt ggc tcg atg ctt ttt acc aaa cag gtt tct gga gcc agg gtt    1200
   Gln Leu Gly Ser Met Leu Phe Thr Lys Gln Val Ser Gly Ala Arg Val
   385                 390                 395                 400
   gtg cct ctt ggg tcc atg caa act gtg agt ggc tat act ttc aga ggc    1248
   Val Pro Leu Gly Ser Met Gln Thr Val Ser Gly Tyr Thr Phe Arg Gly
                   405                 410                 415
   ttt atg tca cat aca aat aat tac cct tgt gct tac ctc aat gct gca    1296
   Phe Met Ser His Thr Asn Asn Tyr Pro Cys Ala Tyr Leu Asn Ala Ala
               420                 425                 430
   tca gcc atc gga atg aar atg cag gat gtg gac ctg ttc ata aac ara    1344
   Ser Ala Ile Gly Met Lys Met Gln Asp Val Asp Leu Phe Ile Asn Xaa
           435                 440                 445
   ctt gac agg tgt tta aag gca gta aga aaa gaa cga agt aaa gag agt    1392
   Leu Asp Arg Cys Leu Lys Ala Val Arg Lys Glu Arg Ser Lys Glu Ser
       450                 455                 460
   gat gac aat tat gac aaa act gaa rat gtg gat att gaa gaa atg gct    1440
   Asp Asp Asn Tyr Asp Lys Thr Glu Xaa Val Asp Ile Glu Glu Met Ala
   465                 470                 475                 480
   tta aaa cta gat aat gta ctt ctt gac aca tac cag gat gct tct tca    1488
   Leu Lys Leu Asp Asn Val Leu Leu Asp Thr Tyr Gln Asp Ala Ser Ser
                   485                 490                 495
   tga                                                                1491
<210> 4
   <211> 496
   <212> PRT
   <213> Homo sapiens
<400> 4
   Ser Arg Arg Glu Ser Gly Trp Cys Arg Arg Leu Thr Cys Gly Arg Ala
    1               5                  10                  15
   Val Arg Pro Ala Ala Arg Met Ser Thr Ser Tyr Gly Cys Phe Trp Arg
               20                  25                  30
   Arg Xaa Xaa Cys Pro Lys Asn Gly Trp Asp Glu Ser Thr Leu Glu Leu
           35                  40                  45
   Phe Leu His Glu Leu Ala Ile Met Asp Ser Asn Asn Phe Leu Gly Asn
        50                  55                  60
   Cys Gly Val Gly Glu Arg Glu Gly Arg Val Ala Ser Ala Leu Val Ala
    65                  70                  75                  80
   Arg Arg His Tyr Arg Phe Ile His Gly Ile Gly Arg Ser Gly Asp Ile
                    85                  90                  95
   Ser Ala Val Gln Pro Lys Ala Ala Gly Ser Ser Leu Leu Asn Lys Ile
               100                 105                 110
   Thr Asn Ser Leu Val Leu Asp Ile Ile Lys Leu Ala Gly Val His Thr
           115                 120                 125
   Val Ala Asn Cys Phe Val Val Pro Met Ala Thr Gly Met Ser Leu Thr
       130                 135                 140
   Leu Cys Phe Leu Thr Leu Arg His Lys Arg Pro Lys Ala Lys Tyr Ile
   145                 150                 155                 160
   Ile Trp Pro Arg Ile Asp Gln Lys Ser Cys Phe Lys Ser Met Ile Thr
                   165                 170                 175
   Ala Gly Phe Glu Pro Val Val Ile Glu Asn Val Leu Glu Gly Asp Glu
               180                 185                 190
   Leu Arg Thr Asp Leu Lys Ala Val Glu Ala Lys Val Gln Glu Leu Gly
           195                 200                 205
   Pro Asp Cys Ile Leu Cys Ile His Ser Thr Thr Ser Cys Phe Ala Pro
       210                 215                 220
   Arg Val Pro Asp Arg Leu Glu Glu Leu Ala Val Ile Cys Ala Asn Tyr
   225                 230                 235                 240
   Asp Ile Pro His Ile Val Asn Asn Ala Tyr Gly Val Gln Ser Ser Lys
                   245                 250                 255
   Cys Met His Leu Ile Gln Gln Gly Ala Arg Val Gly Arg Ile Asp Ala
               260                 265                 270
   Phe Val Gln Ser Leu Asp Lys Asn Phe Met Val Pro Val Gly Gly Ala
           275                 280                 285
   Ile Ile Ala Gly Phe Asn Asp Ser Phe Ile Gln Glu Ile Ser Lys Met
       290                 295                 300
   Tyr Pro Gly Arg Ala Ser Ala Ser Pro Ser Leu Asp Val Leu Ile Thr
   305                 310                 315                 320
   Leu Leu Ser Leu Gly Ser Asn Gly Tyr Lys Lys Leu Leu Lys Glu Arg
                   325                 330                 335
   Lys Glu Met Phe Ser Tyr Leu Ser Asn Gln Ile Lys Lys Leu Ser Glu
               340                 345                 350
   Ala Tyr Asn Glu Arg Leu Leu His Thr Pro His Asn Pro Ile Ser Leu
           355                 360                 365
   Ala Met Thr Leu Lys Thr Leu Asp Glu His Arg Asp Lys Ala Val Thr
       370                 375                 380
   Gln Leu Gly Ser Met Leu Phe Thr Lys Gln Val Ser Gly Ala Arg Val
   385                 390                 395                 400
   Val Pro Leu Gly Ser Met Gln Thr Val Ser Gly Tyr Thr Phe Arg Gly
                   405                 410                 415
   Phe Met Ser His Thr Asn Asn Tyr Pro Cys Ala Tyr Leu Asn Ala Ala
               420                 425                 430
   Ser Ala Ile Gly Met Lys Met Gln Asp Val Asp Leu Phe Ile Asn Xaa
           435                 440                 445
   Leu Asp Arg Cys Leu Lys Ala Val Arg Lys Glu Arg Ser Lys Glu Ser
       450                 455                 460
   Asp Asp Asn Tyr Asp Lys Thr Glu Xaa Val Asp Ile Glu Glu Met Ala
   465                 470                 475                     480
   Leu Lys Leu Asp Asn Val Leu Leu Asp Thr Tyr Gln Asp Ala Ser Ser
                   485                 490                 495